# EUROPEAN PATENT APPLICATION

(11) **EP 4 193 921 A1**
(43) Date of publication of application: **14.06.2023**
(21) Application number: 21212731.0
(22) Date of filing: 07.12.2021
(51) Int. Cl.: A61B 5/087, A61B 5/113

(54) **SYSTEM AND METHOD FOR PROVIDING GUIDANCE DURING SPIROMETRY TEST**

(71) Applicant: Koninklijke Philips N.V., 5656 AG Eindhoven (NL)
(72) Inventor: HIWALE, Sujitkumar, Eindhoven (NL); RADHAKRISHNAN, Ravindranath, Eindhoven (NL); CHAKRABARTI, Biswaroop, Eindhoven (NL); BERA, Deep, Eindhoven (NL)
(74) Representative: Philips Intellectual Property & Standards

(57) **Abstract**

A system and method for providing guidance to a subject and/or operator during a spirometry test. Signals representative of lung sounds and chest/abdominal wall movements of the subject during the spirometry test are received and co-analyzed to provide real-time guidance and error detection via a feedback unit.

## Description

### FIELD OF THE INVENTION

The invention relates to the field of spirometry, and in particular to providing guidance to a subject and/or operator during a spirometry test.

### BACKGROUND OF THE INVENTION

Spirometry is a pulmonary function test widely used for diagnosing and monitoring chronic obstructive pulmonary disease (COPD) and other lung conditions. COPD is a progressive life-threatening disorder characterized by chronic airflow obstruction, which manifests in breathlessness. It is estimated that more than 250 million people suffer from COPD globally, and COPD causes over 3 million deaths each year.

The physical signs of COPD (such as a barrel-shaped chest, a hyperresonant chest percussion note, reduced air sounds across the chest, wheezing, and coarse crackles) are common to many lung conditions and tend to appear late in disease progression. Spirometry is therefore an important tool in obtaining an early and reliable diagnosis of COPD.

Spirometry measures the maximal volume of air that an individual can inspire and expire with maximal effort. During a spirometry test, a subject is asked to take a deepest possible breath and then exhale into the mouthpiece of the spirometer. In order to obtain a useful result, the subject must perform the required maneuver correctly. In practice, subjects tend to find it difficult to perform the maneuver, and there is a wide variability in spirometry results, due to inadequate and variable inspiration (including a delayed start to inspiration and a pause at the peak of inspiration), a premature ending to expiration, and variable effort. It is estimated that only 60% of spirometries in general practice are of acceptable quality with reproducible measurements.

Traditionally, therefore, spirometry is performed under the guidance of an expert operator, who coaches the subject on the required maneuver prior to the procedure and provides constant guidance during the procedure. In order to provide appropriate guidance, the operator must monitor both the volume-pressure loops on the display of the spirometer and the subject simultaneously.

Even with a trained operator, subjects, especially children, often struggle to follow the guidance provided. In particular, many subjects stop being motivated to exhale when they feel nothing more is coming out, even when told to keep blowing.

There is therefore a need for improved guidance during spirometry tests.

### SUMMARY OF THE INVENTION

The invention is defined by the claims.

According to examples in accordance with an aspect of the invention, there is provided a processing system for providing guidance to a subject and/or operator during a spirometry test.

The processing system is configured to: receive, from at least one microphone, a first signal responsive to respiratory sounds of the subject; receive, from at least one sensor mounted on the subject, a second signal responsive to a movement, orientation and/or position of a chest wall and/or abdominal wall of the subject; generate, by processing the first and second signals, guidance information for adjusting one or more characteristics or actions of the subject for the spirometry test; and control a feedback unit to provide a user-perceptible output representative of the generated guidance information.

The combination of respiratory sounds and chest wall movements enables the detection of any anomalies during the spirometry test, and therefore allows appropriate guidance to be provided to the subject and/or operator. The inventors have recognized that these signals provide information that cannot be identified from the spirometry recording itself. This allows the subject to be monitored during the spirometry test more reliably than an operator may be able to by observing the subject, allowing spirometry tests to be conducted by less skilled operators.

The guidance may be provided in the form of, for example, haptic, audio and/or visual feedback. Thus, the user perceptible output may be a haptic, audio and/or visual user-perceptible output.

The processing system may be configured to generate guidance information by performing a process comprising at least one of: determining a current posture of the subject; determining a difference between the current posture of the subject and a desired posture; detecting a start of inspiration; detecting a peak of inspiration; detecting an end of inspiration; determining a rate of chest wall displacement; detecting outward chest displacement; determining a maximum value of outward chest displacement; determining a rate of abdominal wall displacement; determining an amplitude of abdominal wall displacement; detecting a start of expiration; determining a duration between the peak of inspiration and a start of expiration; determining a maximum inward chest displacement; detecting a peak of expiration; detecting an end of expiration; comparing the received signals with reference signals; comparing the received signals with previous signals from the subject; and/or detecting an early termination of breath.

It is important that the subject has a correct posture in order to perform the spirometry maneuver correctly. In routine practice, an operator will ensure that the subject has assumed the correct posture at the start of the spirometry test, but subjects tend to change their posture during the test and it is difficult for the operator to monitor the subject's posture while carrying out the test. The use of position sensors allows the subject's posture to be continuously monitored throughout the test.

Respiratory sounds and chest/abdominal wall movements may be used to detect respiratory phases, and in particular to detect the start, peak and end of inspiration and expiration. This information may be used to detect a slow start to inspiration, a pause at the peak of inspiration, and an early termination of breath. These are common problems in spirometry, and can lead to incorrect measurements of peak expiratory flow and forced expiratory volume in the first second. Currently, these are detected based only on an operator's visual observation of the subject; however, this requires a highly-skilled operator, and it is not always possible to detect these errors from visual observation even with an expert operator. The use of information from microphone(s) and position sensor(s) allows these errors to be detected more reliably.

The microphone and position sensor data may also be used to detect other common errors, such as coughing, jerky movements, and subject fatigue, by comparing the data obtained during a maneuver with that obtained during previous maneuvers and/or reference data.

The guidance information may comprise at least one of: an indication of whether or not the subject has a desired posture; feedback to guide the subject to a desired posture; an indication to prompt the subject to start inspiration; an indication of a detection of a slow start to inspiration; an indication of a detection of a pause at a peak of inspiration; an indication to prompt the subject to start expiration; an indication of a slow start to expiration; an indication to guide the subject to continue to exhale; an indication of a detection of an end of expiration; an indication of a detection of a cough; an indication of a detection of subject fatigue; and/or an indication of a detection of an early termination of breath.

For example, the subject may be guided to a correct posture by the use of vibrator elements on the subject's chest/back that vibrate if the subject needs to correct their posture. The position of the vibrator elements may be used to indicate how the subject needs to correct their posture: for example, a vibrator element on a particular side of the subject's chest/back may vibrate to indicate that the subject should correct their posture in that direction. Alternatively, the operator may be informed that the subject's posture is incorrect.

The subject may, for example, be instructed to start maximal inspiration when it is detected that the subject has a correct posture for performing the maneuver. An instruction to start inspiration may, for example, be provided by a vibrator element positioned on the subject's body, or be communicated to the operator.

Detection of errors such as a slow start to inspiration, a pause at the peak of inspiration, an early termination of breath, coughing, jerky movements, and subject fatigue may be communicated to the subject and/or operator so that incorrect maneuvers may be stopped as soon as the error is detected and to allow spirometry data collected during incorrect maneuvers to be discarded.

In some embodiments, the processing system is further configured to receive a spirometry signal of the subject from a spirometer.

In some embodiments, the processing system is further configured to analyze the spirometry signal to detect whether the spirometry signal has reached a plateau and/or to calculate a forced expiratory time.

In this way, the conventional parameters in spirometry data used to detect an end of maximal expiration may be combined with the information provided by the microphone(s) and position sensor(s) to detect an end of expiration more reliably than by using either alone.

In some embodiments, the spirometry signal is representative of a plurality of maneuvers performed by the subject, each maneuver comprising a different inspiration and expiration; and the processing system is further configured to select a maneuver from the plurality of maneuvers that most closely matches a predetermined desired maneuver by processing the spirometry signal and the received first and second signals.

A desired maneuver may be the maneuver most suitable or most appropriate for final analysis. For example, a desired maneuver may be a maneuver during which the subject had a desired posture, that is devoid of recording anomalies, and that meets acceptability and usability criteria of standard guidelines, such as the American Thoracic Society and European Respiratory Society Technical Statement. Conventionally, a maneuver for final analysis is selected solely on the basis of spirometry data, which may be misleading if errors occurred during any of the maneuvers. By considering the information provided by the microphone(s) and/or position sensor(s) in addition to the spirometry data, errors may be taken into account when selecting the maneuver to use for final analysis.

The processing system may be configured to select the maneuver by: analyzing, for each maneuver in the spirometry signal, the first and second signals received during the maneuver to identify whether any errors occur in the spirometry test performed during the maneuver; defining a subset of the plurality of maneuvers, wherein the subset comprises only those maneuvers for which no errors are identified by the analysis; and selecting the maneuver from the subset based on one or more characteristics of the spirometry signal.

In this way, maneuvers containing errors may be identified and discarded before selecting a maneuver to use for final analysis.

In some embodiments, the processing system is further configured to provide a diagnosis of respiratory pathology based on the spirometry signal and the received first and second signals.

This recognizes that processing the spirometry signal and auscultation data simultaneously enables the differentiation of respiratory pathologies. Spirometry provides information on pulmonary function test for both lungs together, but does not provide information on specific pathologies, while auscultation provides information on local pathologies.

In some embodiments, the processing system is further configured to: receive a third signal responsive to an electrical activity of a muscle of the subject from at least one electromyography sensor; and generate the guidance information by processing the first, second and third signals.

This information may be used in combination with the information from the microphone(s) and position sensor(s) to provide the most appropriate guidance to the subject and/or operator.

The processing system may be configured to generate the guidance information by performing a process comprising: determining a peak force and/or total force generated by the muscle based on the third signal; and detecting muscle fatigue based on the determined peak force and/or total force generated by the muscle.

This information may, for example, be used in assessing a quality of acquired spirometry records, and may be particularly useful in discarding low quality maneuvers when selecting a best maneuver (i.e. a maneuver suitable for use in final analysis).

The processing system may be further configured to determine a total muscle mass used during a maneuver based on the third signal.

A higher muscle recruitment indicates a more forceful effort. The total muscle mass used may therefore be used in combination with the spirometry data to select a best maneuver of a plurality of maneuvers.

There is also proposed a system for providing guidance to a subject and/or operator during a spirometry test. The system comprises: at least one microphone configured to obtain a signal responsive to respiratory sounds of the subject; at least one sensor mounted on the subject configured to obtain a signal responsive to a movement, orientation and/or position of a chest wall and/or abdominal wall of the subject; the processing system described above, configured to receive the first signal from the at least one microphone and the second signal from at least one position sensor; and a feedback unit, configured to provide a user-perceptible output representative of the generated guidance information based on an instruction from the processing system.

The at least one sensor mounted on the subject may comprise at least one inertial measurement unit, at least one accelerometer, and/or at least one strain gauge.

According to another aspect of the invention, there is provided a computer-implemented method for providing guidance to a subject and/or operator during a spirometry test, the computer-implemented method comprising: receiving, from at least one microphone, a first signal responsive to respiratory sounds of the subject; receiving, from at least one sensor mounted on the subject, a second signal responsive to a movement, orientation and/or position of a chest wall and/or abdominal wall of the subject; generating, by processing the first and second signals, guidance information for adjusting one or more characteristics or actions of the subject for the spirometry test; and controlling a feedback unit to provide a user-perceptible output representative of the generated guidance information.

There is also proposed a computer program product comprising computer program code means which, when executed on a computer device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system for providing guidance to a subject and/or operator during a spirometry test, according to an embodiment of the invention;
Figure 2 illustrates a method for providing posture guidance throughout a spirometry test, according to an embodiment of the invention;
Figure 3 illustrates example IMU data from a chest surface of a single subject for three different breathing patterns;
Figure 4 illustrates an example decision tree that may be used to provide guidance during inspiration, according to an embodiment of the invention;
Figure 5 illustrates an example decision tree that may be used to provide guidance during expiration, according to an embodiment of the invention;
Figure 6 illustrates example IMU (roll in degrees) data from a chest of a subject over multiple respiratory maneuvers;
Figure 7 illustrates a system for providing guidance to a subject and/or operator during a spirometry test, according to an embodiment of the invention;
Figure 8 illustrates an example assessment of respiratory pathologies based on spirometry and auscultation data; and
Figure 9 illustrates a computer-implemented method for providing guidance to a subject and/or operator during a spirometry test, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for providing guidance to a subject and/or operator during a spirometry test. Signals representative of lung sounds and chest/abdominal wall movements of the subject during the spirometry test are received and co-analyzed to provide real-time guidance and error detection via a feedback unit.

Embodiments are at least partly based on the realization that these signals may be used to determine information that cannot be determined from a spirometry recording, such as a posture of the subject.

Illustrative embodiments may, for example, be employed in digital physical examination systems, respiratory monitoring systems, and spirometry systems.

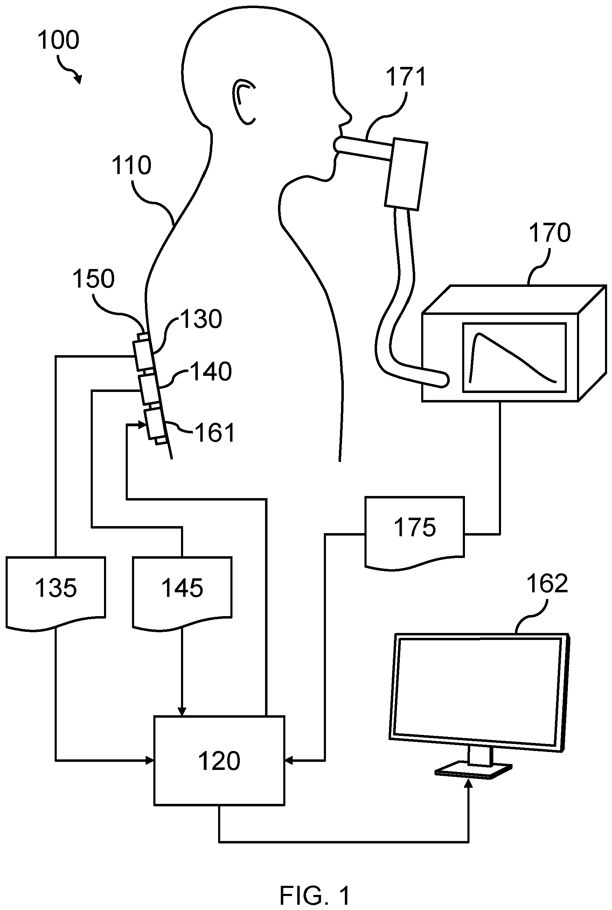

Figure 1 illustrates a system 100 for providing guidance to a subject 110 and/or operator (not shown) during a spirometry test, according to an embodiment of the invention. The system comprises a plurality of sensors, a processing system 120, and a feedback unit. The processing system is, itself, an embodiment of the invention.

The plurality of sensors comprises at least one microphone 130 configured to obtain a signal responsive to respiratory sounds of the subject 110 and at least one sensor 140 mounted on the subject configured to obtain a signal responsive to a movement, orientation and/or position of a chest wall and/or abdominal wall of the subject. The at least one microphone may comprise a simple microphone, or a microphone array may be used for improved localization. The at least one sensor 140 may comprise, for example, at least one inertial measurement unit (IMU), at least one accelerometer, at least one strain gauge and/or any other sensor suitable for obtaining a signal responsive to a movement, orientation and/or position of a chest wall and/or abdominal wall. In some examples, the plurality of sensors may further comprise at least one sensor for obtaining a signal responsive to a movement, orientation and/or position of the subject's head.

In the illustrated embodiment, the at least one microphone 130 and at least one sensor 140 are integrated into a patch 150 placed on the subject's back. A patch with the at least one microphone and at least one sensor may additionally or alternatively be placed on the subject's chest and/or abdomen. A single patch may be positioned such that it covers both sides of the chest wall, or two separate patches may be placed on different sides of the chest wall such that they cover corresponding areas. Alternatively, the at least one microphone and at least one sensor may be integrated into a belt wrapped around the subject's chest or abdomen or otherwise coupled to the subject (e.g. with adhesive or strapping/webbing).

The processing system 120 receives a first signal 135 responsive to respiratory sounds of the subject 110 from the at least one microphone 130, and a second signal 145 responsive to a movement, orientation and/or position of a chest wall and/or abdominal wall of the subject from the at least one sensor 140. The processing system then processes the first and second signals to generate guidance information for adjusting one or more characteristics or actions of the subject for the spirometry test and controls the feedback unit to provide a user-perceptible output representative of the generated guidance information.

The step of processing the first signal 135 and second signal 145 to generate guidance information may comprise one or more of: determining a current posture of the subject; determining a difference between the current posture of the subject and a desired posture; detecting a start of inspiration; detecting a peak of inspiration; detecting an end of inspiration; determining a rate of chest wall displacement; detecting outward chest displacement; determining a maximum value of outward chest displacement; determining a rate of abdominal wall displacement; determining an amplitude of abdominal wall displacement; detecting a start of expiration; determining a duration between the peak of inspiration and the start of expiration; determining a maximum inward chest displacement; detecting a peak of expiration; detecting an end of expiration; comparing the received signals with reference signals; comparing the received signals with previous signals from the subject; and/or detecting an early termination of breath.

The processing system 120 may use any appropriate algorithm(s) and/or machine-learning method(s) (e.g. decision trees, logistical regressions, support vector machines, random forests and/or neural networks) to generate guidance information. For example, an algorithm or combination of algorithms may be trained on a large dataset of data representative of respiratory sounds and chest/abdominal wall movements during spirometry maneuvers to detect abnormalities in the first signal 135 and/or second signal 145.

The generated guidance information may comprise one or more of: an indication of whether or not the subject has a desired posture; feedback to guide the subject to a desired posture; an indication to prompt the subject to start inspiration; an indication of a detection of a slow start to inspiration; an indication of a detection of a pause at a peak of inspiration; an indication to prompt the subject to start expiration; an indication of a detection of a slow start to expiration; an indication to guide the subject to continue to exhale; an indication of a detection of an end of expiration; an indication of a detection of a cough; an indication of a detection of subject fatigue; and/or an indication of a detection of an early termination of breath.

The generated guidance information may be directed towards the subject 110, the operator or both. In some examples, the processing system may generate separate guidance information for the subject and operator, and guidance information for the operator may be provided via a separate device to a device delivering guidance information to the subject.

The user-perceptible output representative of the generated guidance information is provided to the subject 110 and/or operator by the feedback unit based on an instruction from the processing system 120.

In Figure 1, the feedback unit comprises a vibrator 161 and an audio-visual display device 162. The vibrator is integrated into the patch 150 and provides guidance information to the subject in the form of haptic feedback. The display device provides visual and/or audio guidance to the subject and/or operator. However, the feedback unit is not limited to a vibrator and/or a display device, and any device suitable for providing a user-perceptible output (e.g. a haptic, audio or visual indicator), such as a speaker or a light, may be used to provide the user-perceptible output representative of the generated guidance information. The user-perceptible output may comprise, for example, one or more of a vibration, a sound, a textual display (e.g. an instruction or a notification) and/or an image.

In some examples, the vibrator 161 may be used to provide a plurality of user-perceptible outputs, each representative of different guidance information. In these examples, different vibration signals (e.g. having different intensities, different patterns and/or different durations) may be used to allow the subject 110 to differentiate between different feedbacks. Additionally or alternatively, the subject may be provided with an audio and/or visual indication (e.g. on the display device 162) alongside the indication from the vibrator, with the vibration being used to prompt the subject to pay attention to the audio and/or visual indication.

During the spirometry test, the subject 110 performs at least one spirometry maneuver, in which they take a deepest possible (for the subject) breath and then exhale into a mouthpiece 171 of a spirometer 170 until all the air (i.e. as much air as possible for the subject) has been expelled from their lungs. The spirometer measures the volume of air exhaled by the subject.

In order to perform a spirometry maneuver correctly (i.e. to achieve clinically useful results from the spirometry test, e.g. according to medical guidelines), the subject 110 should have a suitable posture (upright with the head slightly elevated) throughout the maneuver. In some examples, the system 100 monitors the posture of the subject and provides feedback to guide the subject to a posture that is suitable for performing the spirometry maneuver (i.e. a desired posture).

The processing system 120 may determine a current posture of the subject 110 based on the second signal 145 responsive to a movement, orientation and/or position of a chest wall and/or abdominal wall of the subject (and optionally further based on a signal responsive to a movement, orientation and/or position of the subject's head).

Methods for determining posture based on such a signal are well known and will be apparent to the skilled person. In particular, the use of IMUs, accelerometers, and/or strain gauges for monitoring posture is well known: see, for example, Cao et al. (2017), "Realtime multi-person 2D pose estimation using part affinity fields", arXiv:1611.08050 [cs]; Nguyen et al. (2013), "Novel automatic posture detection for in-patient care using IMU sensors", 6th IEEE Conference on Robotics, Automation and Mechatronics (RAM), pp. 31-36; Fahrenberg et al. (1997), "Assessment of posture and motion by multichannel piezoresistive accelerometer recordings", Psychophysiology, 34(5):607-612; and Donatell et al. (2005), "A simple device to monitor flexion and lateral bending of the lumbar spine", IEEE Transactions on Neural Systems and Rehabilitation Engineering, 13(1): 18-23.

The processing system 120 may determine whether the current posture of the subject 110 is suitable for spirometry by comparing the current posture with a predetermined desired posture. For example, the processing system may determine a difference between a value of one or more parameters of a current posture and a corresponding value of each of the one or more parameters of a predetermined desired posture. The one or more parameters may, for instance, comprise an orientation in a left-right direction and an orientation in a forward-backward direction. Other suitable parameters for comparing a current and a desired posture will be apparent to the skilled person. The desired posture may represent a clinically recommended posture for a spirometry test (e.g. as indicated in literature such as medical guidelines and/or by a suitable trained professional).

The processing system 120 may determine that the current posture of the subject 110 is not a desired posture in response to the determined difference for any of the one or more parameters exceeding a predetermined threshold. If the processing system determines that the current posture is not a desired posture, the processing system may generate guidance information comprising an indication that the subject does not have a desired posture and/or feedback to guide the subject to a desired posture.

For example, the processing system 120 may instruct the display device 162 to provide an audio and/or visual indication that the subject 110 does not have a desired posture. The audio and/or visual indication may further include information about the subject's current posture and/or an indication of a direction in which the subject should adjust their posture in order to achieve a suitable posture. This allows an operator to be alerted to the fact that the subject has an unsuitable posture without requiring the operator to constantly observe the subject. The operator may then instruct the subject on how to correct their posture.

Additionally or alternatively, the processing system 120 may instruct the vibrator 161 to provide an indication that the subject 110 does not have a desired posture. In some examples, the vibrator may simply vibrate to alert the subject to the fact that their posture is not suitable for spirometry maneuvers. In other examples, a plurality of vibrators may be placed on the subject in different positions, and which vibrator(s) provide the indication may depend on a direction in which a subject should adjust their posture in order to achieve a suitable posture. For example, if processing system determines that the subject is leaning too far forward (e.g. based on a forward orientation exceeding a predetermined threshold), the processing system may control a vibrator positioned on the subject's front to vibrate, or the processing system may control a vibrator positioned on the subject's left side to vibrate in response to a determination that the subject is leaning too far to the left (e.g. based on a leftward orientation exceeding a predetermined threshold).

In some examples, the system 100 may further comprise a user-input device (not shown in Figure 1) to allow the operator to control a feedback provided to the subject 110. In other words, the processing system 120 may be configured to control the feedback unit to provide a user-perceptible output representative of the generated guidance information to the operator, receive a user-input from the operator, and control the feedback unit to provide a user-perceptible output to the subject based on the received user-input. For example, the operator may select which of a plurality of vibrators placed in different positions on the subject they want to be activated responsive to an indication that the subject does not have a desired posture, and the processing system may control the selected vibrator(s) to vibrate.

The processing system 120 may continue to determine (i.e. iteratively determine) a current posture of the subject 110 and provide feedback to guide the subject to correct their posture until the processing system determines that the subject has a desired posture (e.g. until the determined difference between a value of one or more parameters of a current posture and a corresponding value of each of the one or more parameters of a predetermined desired posture does not exceed a predetermined threshold for any of the one or more parameters). For example, the processing system may be configured to determine a current posture and provide feedback at predetermined intervals, or the processing system may continuously determine a current posture and provide feedback only when a current posture differs from a previous current posture.

In some examples, the processing system 120 may generate guidance information comprising an indication to prompt the subject 110 to start inspiration in response to a determination that the subject has a desired posture. The indication may be provided directly to the subject, or the indication may be provided to the operator, who may then instruct the subject to start inspiration. For example, the processing system may control the display device 162 to provide an audio and/or visual indication that the subject now has a suitable posture for performing a spirometry maneuver, and/or control the vibrator 161 to vibrate to prompt the subject to start inspiration.

In some examples, the processing system 120 may continue to monitor the posture of the subject 110 as the subject performs the spirometry maneuver. For example, the processing system may determine a current posture of the subject continuously or at predetermined intervals, and provide an indication to the subject and/or operator in response to a determination that the current position no longer corresponds to a desired position.

In particular, the processing system 120 may detect any abnormal movements, such as sudden jerks or coughs, that occur during the maneuver. These movements can lead to measurement errors. Alerting the subject and/or operator to the detection of such movements allows maneuvers in which they occur to be abandoned as soon as they are detected. This reduces time wasted by completing maneuvers for which the recorded spirometry data cannot be used and reduces the likelihood of a subject becoming fatigued before they are able to perform a required number of correct maneuvers.

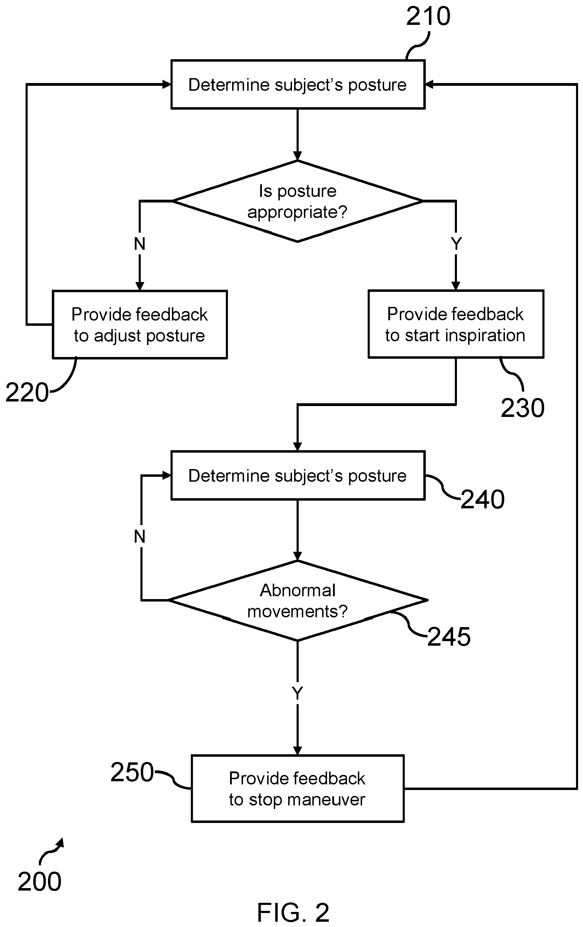

Figure 2 illustrates a method 200 for providing posture guidance during/throughout a spirometry test, according to an embodiment of the invention.

The method begins, at the start of the spirometry test, with step 210, in which a subject's current posture is determined.

If the subject's current posture is determined not to be appropriate for spirometry (i.e. if the subject does not have a desired posture), the method proceeds to step 220, in which feedback is provided to the subject and/or an operator to guide the subject to a desired posture.

Steps 210 and 220 are repeated until the subject's current posture is determined to be appropriate for spirometry, when the method proceeds to step 230, in which feedback is provided to the subject and/or operator to prompt the subject to start inspiration.

At step 240, the subject's posture continues to be determined unless an abnormal movement is detected (e.g. by step 245), in which case, the method proceeds to step 250.

At step 250, feedback is provided to the subject and/or operator to inform them that an error has been detected and a current maneuver should be abandoned. The method then returns to step 210, determining the subject's current posture, and guiding the subject to adjust their posture or prompting the subject to begin a new maneuver according to whether or not the subject has a desired posture.

In some examples, step 210-230 may be omitted. For instance, an operator may (before the subject begins a spirometry maneuver) position the subject in a suitable posture. In these examples, step 250 may provide a recommendation to restart the spirometry maneuver, before reverting back to step 240 (e.g. when the spirometry maneuver has been restarted - e.g. as indicated by a user input or through monitoring of exhalation by the subject).

To correctly perform a spirometry maneuver, a subject is required to achieve maximal inflation; that is, the subject must inspire to a maximum possible extent. Many subjects will not have achieved this before, and they may find it uncomfortable. Common problems during spirometry tests include a slow start to inspiration and a pause (e.g. 4-6 seconds in duration) at a peak of inspiration before beginning expiration. These problems can cause errors in spirometry measurements, such as reductions in the peak expiratory flow (PEF) and forced expiratory volume in the first second (FEV1), and are not always apparent from a visual observation of the subject.

In some examples, the system 100 may detect a slow start to inspiration and/or a pause at the peak of inspiration, and provide appropriate guidance to the subject and/or operator.

Returning to Figure 1, both heart and respiratory sounds are transmitted through the chest wall of the subject 110, and therefore both are detected by the at least one microphone 130. The processing system 120 may process the first signal 135 from the at least one microphone to filter out heart sounds in the first signal. Methods for differentiating between heart and respiratory sounds are well known (see, for example, Gnitecki and Moussavi (2007), "Separating heart sounds from lung sounds", IEEE Engineering in Medicine and Biology Magazine, 26(1):20-29).

In examples in which the at least one microphone 130 comprises a microphone array, the microphone array may be used to determine locations of ribs and intercostal spaces. One appropriate mechanism for detecting ribs and intercostal spaces is provided by the European patent application having publication no. EP3701876. Based on this determination, the first signal 135 may be configured to comprise only sounds received by microphones in intercostal spaces (e.g. by deactivating rib-aligned microphones in the microphone array) to improve an accuracy of the first signal.

The respiratory information carried by the first signal 135 has two components, corresponding to inspiratory sounds and expiratory sounds. These sounds have distinct characteristics: inspiratory sounds have a rustling quality generated by turbulent airflow within the lobes of the lungs, while expiratory sounds are more sibilant. During expiration, the sound becomes softer as the air flows within larger airways.

The processing system 120 may therefore identify inspiratory sounds and expiratory sounds in the first signal 135 based on one or more characteristics of the first signal. Methods for distinguishing between inspiratory and expiratory sounds in a signal responsive to respiratory sounds are known: see, for example, Jácome et al. (2019), "Convolutional neural network for breathing phase detection in lung sounds", Sensors, 19(8):1798.

The chest wall and abdomen wall of the subject 110 move outward during inspiration and inward during expiration. The second signal 145 responsive to a movement, orientation and/or position of a chest wall and/or abdominal wall of the subject may therefore also be used to detect inspiration and expiration. The use of measurements of chest wall movements and/or abdominal wall movements to detect respiratory phases is known: see, for example, Cesareo et al. (2019), "Assessment of breathing parameters using an inertial measurement unit (IMU)-based system", Sensors, 19(1):88; and Mojtaba et al. (2014), "Accelerometer-based method for extracting respiratory and cardiac gating information for dual gating during nuclear medicine imaging", International Journal of Biomedical Imaging, Article ID 690124.

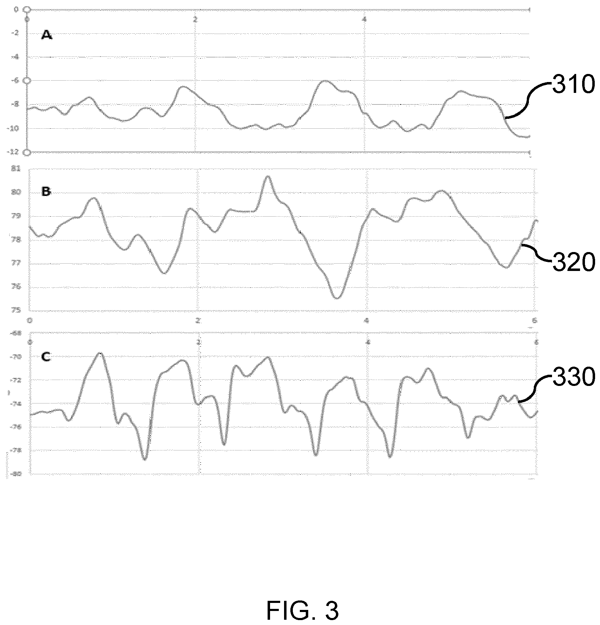

Figure 3 illustrates example IMU data (IMU roll in degrees against time in seconds) from a chest surface of a single subject for three different breathing patterns. Graph 310 illustrates a normal breathing pattern, graph 320 illustrates slow breathing with maximal inflation, and graph 330 illustrates fast breathing with maximal inflation. As Figure 3 shows, each breathing pattern has a characteristic signal. A classifier may be built using established signal processing and algorithm-based techniques to identify a breathing type pattern from IMU data (such as the second signal 145). This information may be used to detect a start, peak and end of inspiration and expiration, and to detect errors such as a slow start to inspiration and a pause at a peak of inspiration.

Returning to Figure 1, the processing system 120 may synchronize and process the first signal 135 and/or second signal 145 to detect the start and end of inspiration, and the start and end of expiration using any of the methods outlined above.

The second signal 145 may also be used to measure the rate at which the chest and/or abdominal wall expands during inspiration and contracts during expiration (the rate of chest and/or abdominal wall displacement). The rate of chest and/or abdominal wall displacement is related to both the speed and vigor of inspiration and expiration, and may therefore be used to detect a slow start to inspiration.

For example, the processing system 120 may detect a slow start to inspiration in response to a determination that a rate of chest and/or abdominal wall displacement during inspiration is below a predetermined threshold. A suitable value for the threshold may be derived based on experimental data representative of a rate of chest and/or abdominal wall displacement during spirometry recording. Different thresholds may be used for subjects having different characteristics (e.g. sex, age and ethnicity).

In response to detecting a slow start to inspiration, the processing system 120 may generate guidance information comprising an indication of a detection of a slow start to inspiration. The processing system 120 may control the feedback unit 161, 162 to provide an indication of a detection of a slow start to inspiration. This allows maneuvers having a slow start to inspiration to be terminated early, rather than continuing with a maneuver that cannot be used in spirometry analysis.

For example, the processing system 120 may instruct the display device 162 to provide an audio and/or visual indication that a slow start to inspiration has been detected and/or instruct the vibrator 161 to vibrate to indicate that a slow start to inspiration has been detected. If the guidance information is provided to the operator, the operator may instruct the subject to abandon the maneuver, or cause the vibrator to be activated using the user input device in order to alert the subject.

The second signal 145 may also be used to measure outward chest and/or abdominal wall displacement during inspiration. Outward chest and/or abdominal wall displacement has a maximum value for a given maneuver at the peak of inspiration. The outward chest and/or abdominal wall displacement may therefore be used to detect a peak of inspiration. A maximum value of outward chest and/or abdominal wall displacement for the subject 110 may be determined based on population data for different groups (e.g. based on sex, age, etc.).

The rate of chest and/or abdominal wall displacement and/or the first signal 135 may additionally or alternatively be used to detect a peak of inspiration. For example, a peak of inspiration may be detected in response to a determination that a rate of chest and/or abdominal wall displacement during inspiration is below a predetermined threshold (which may be a different threshold to the threshold used to detect a slow start to inspiration, e.g. a value close to zero), and/or in response to a pause in inspiratory sounds in the first signal 135.

In order to distinguish between a low rate of chest and/or abdominal wall displacement due to a slow start to inspiration and a low rate due to reaching a peak of inspiration, the thresholds used to detect a slow start to inspiration and a peak of inspiration may further be defined with respect to time. For example, a time cut-off may be used to determine which threshold to apply: a rate of chest and/or abdominal wall displacement below both thresholds may indicate a slow start to inspiration if it occurs before the time cut-off and a peak of inspiration otherwise.

Additionally or alternatively, other characteristics may be used in combination with the rate of chest and/or abdominal wall displacement to distinguish between a slow start to inspiration and a peak of inspiration. For instance, a rate of chest and/or abdominal wall displacement below both thresholds may indicate a peak of inspiration if a maximum value of outward chest displacement has been reached and a slow start to inspiration otherwise.

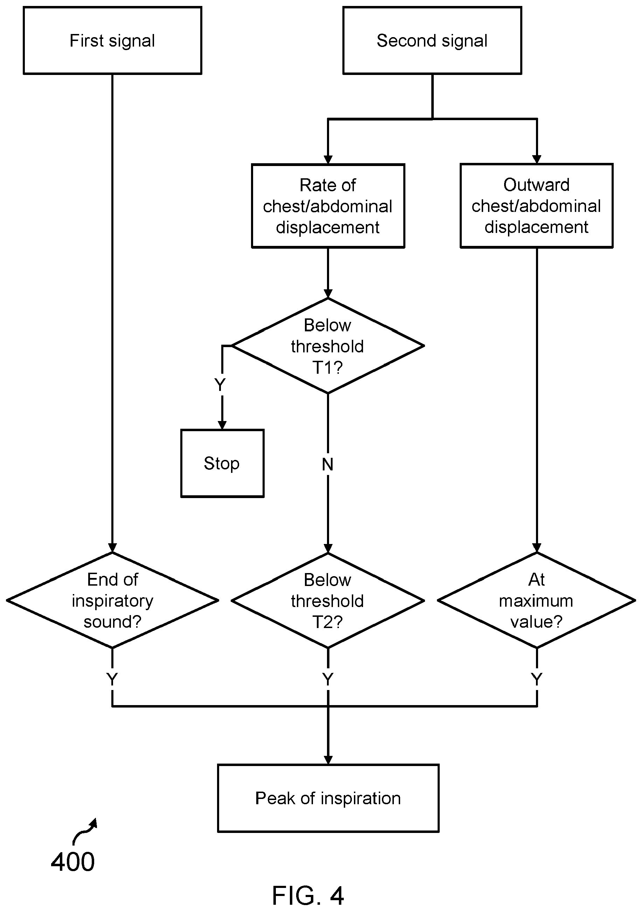

The parameters described above for detecting a slow start to inspiration and a peak of inspiration are summarized in Figure 4, which illustrates an example decision tree 400 that may be used to provide guidance during inspiration, according to an embodiment of the invention.

A slow start to inspiration is detected in response to a determination that a rate of chest and/or abdominal wall displacement is below a first threshold, T1, and guidance information is provided to prompt the subject to stop. A peak of inspiration is detected in response to a determination that a rate of chest and/or abdominal wall displacement is below a second threshold, T2, a determination that an outward chest and/or abdominal wall displacement has reached a maximum value, and/or a determination that inspiratory sounds have ceased.

Returning to Figure 1, a detection of a peak of inspiration may be used to detect a pause at the peak of inspiration. For example, the processing system 120 may detect a pause at the peak of inspiration in response to a determination that a duration between a peak of inspiration and a start of expiration (or a duration since a peak of inspiration without a start of expiration being detected) exceeds (or will exceed) a predetermined threshold (e.g. 2 seconds).

A duration between a peak of inspiration and a start of expiration may, for example, be determined based on a duration between a time at which a maximum value of outward chest and/or abdominal wall displacement is reached and a time at which an inward chest and/or abdominal wall displacement starts, and/or based on a duration between a time at which inspiratory sounds in the first signal 135 cease and a time at which expiratory sounds start.

In response to detecting a pause at the peak of inspiration, the processing system 120 may generate guidance information comprising an indication of a detection of a pause at the peak of inspiration and/or an indication to prompt the subject 110 to start expiration. For example, the processing system may control the display device 162 to provide an audio and/or visual indication that a pause at the peak of inspiration has been detected to the operator. The operator may instruct the subject to start expiration, or cause the vibrator 161 to be activated using the user input device in order to prompt the subject to start expiration. In another example, the processing system may control the vibrator to provide an indication to prompt the subject to start expiration.

After feedback regarding a pause at the peak of inspiration has been provided to the subject and/or operator, the processing system 120 may continue to measure a duration since the peak of inspiration was reached until the processing system detects a start of expiration. In response to a determination that the duration has exceeded a predetermined threshold (e.g. 2 or 4 seconds) without expiration having started, the processing system may generate guidance information comprising an indication that a pause at the peak of inspiration has lasted too long, to prompt the subject and/or operator to stop the maneuver. This allows maneuvers that cannot provide useful spirometry data due to the length of the pause at the peak of inspiration to be abandoned as soon as it is detected that the maneuver is not suitable for use in spirometry analysis.

In a spirometry maneuver, a subject is required to expire fully and forcefully. The operator typically uses spirometry data to detect when a subject has reached maximal expiration. Maximal expiration is detected in spirometry data based on a determination that a plateau has been reached in the spirometry signal or that a forced expiratory time (FET) has reached 15 seconds. However, these parameters are not useful indicators of maximal expiration in some subjects with restrictive lung disease or in young subjects who have high elastic recoil and can empty their lungs quickly. In addition to this, subjects often perform the expiratory phase of the maneuver incorrectly. Common errors during the expiratory phase of a spirometry maneuver include early termination and incomplete exhalation. It is not necessarily obvious to an operator that one of these errors has occurred based on the spirometry data alone.

In some examples, the system 100 may provide guidance during expiration to encourage the subject 110 to continue exhaling if maximum expiration has not been reached, provide an indication that maximum expiration has been reached, and/or detect an error during expiration.

The processing system 120 may detect a start and end of expiration based on the first signal 135 and second signal 145 as described above.

A slow start to expiration may be detected based on a rate of chest and/or abdominal wall displacement in a similar way to the detection of a slow start to inspiration described above. For example, the processing system 120 may detect a slow start to expiration in response to a determination that a rate of chest and/or abdominal wall displacement during expiration is below a predetermined threshold. As with the threshold for detecting a slow start to inspiration, a suitable value for the threshold may be derived based on experimental data, and different thresholds may be used for subjects having different characteristics (e.g. sex, age and ethnicity).

In response to detecting a slow start to expiration, the processing system 120 may generate guidance information comprising an indication of a detection of a slow start to expiration, and control the vibrator 161 and/or the display device 162 to provide an indication that a slow start to expiration has been detected. This allows a poor effort to be terminated early, rather than continuing with a tiring forced expiration.

At a peak of expiration, the rate of chest and/or abdominal wall displacement will become very slow (almost static), and an inward contraction of the chest and/or abdominal wall will reach a maximum. The rate of chest and/or abdominal wall displacement, and/or the inward chest and/or abdominal wall displacement may therefore be used to detect an end of expiration.

For example, the processing system 120 may detect an end of expiration in response to a determination that a rate of chest and/or abdominal wall displacement during expiration is below a predetermined threshold (which may be a different threshold to the threshold used to detect a slow start to expiration, e.g. close to zero), and/or in response to a determination that an inward chest and/or abdominal wall displacement has reached a maximum value. As with the maximum outward chest and/or abdominal wall displacement, a maximum value of inward chest and/or abdominal wall displacement for the subject 110 may be determined based on population data for different groups (e.g. based on sex, age, etc.).

A low rate of chest and/or abdominal wall displacement due to a slow start to expiration may be distinguished from a low rate due to reaching an end of expiration as described above with reference to the corresponding thresholds during inspiration. For example, a time cut-off may be used to determine whether a rate of chest and/or abdominal wall displacement indicates a slow start to expiration or an end of expiration when the rate is below both the threshold for detecting a slow start to expiration and the threshold for detecting an end of expiration, and/or other characteristics (such as an inward chest displacement) may be used in combination with the rate of chest and/or abdominal wall displacement.

The first signal 135 may also be used to detect an end of expiration. For example, the processing system 120 may detect an end of expiration in response to a pause in expiratory sounds in the first signal 135.

In some examples, the processing system 120 receives a spirometry signal 175 from the spirometer 170. The spirometry signal is representative of the volume of air expired by the subject 110.

The processing system 120 may analyze the spirometry signal 175 to detect whether the spirometry signal has reached a plateau and/or to calculate a forced expiratory time (FET). A plateau is generally considered to have been reached if a change in volume is below a predetermined threshold for a predetermined duration. These are the conventional parameters used to detect maximum expiration, and methods for analyzing the spirometry signal to determine these parameters will be apparent to the skilled person.

The processing system 120 may use these parameters (and/or other suitable parameters of the spirometry signal 175) in combination with the rate of chest and/or abdominal wall displacement, the inward chest and/or abdominal wall displacement, and the respiratory sounds from the first signal 135 to detect maximum expiration.

In response to detecting maximum expiration, the processing system 120 may generate guidance information comprising an indication of a detection of maximum expiration, and control the vibrator 161 and/or the display device 162 to provide an indication of a detection of maximum expiration to the subject 110 and/or operator. Subjects are not always aware when maximum expiration has been reached; indeed, many feel that they have no more air to breathe out while they are still expiring. An indication that maximum expiration has been reached allows a subject to know when they can stop trying to exhale. The subject may be instructed to continue to exhale until an indication of a detection of maximum expiration is provided.

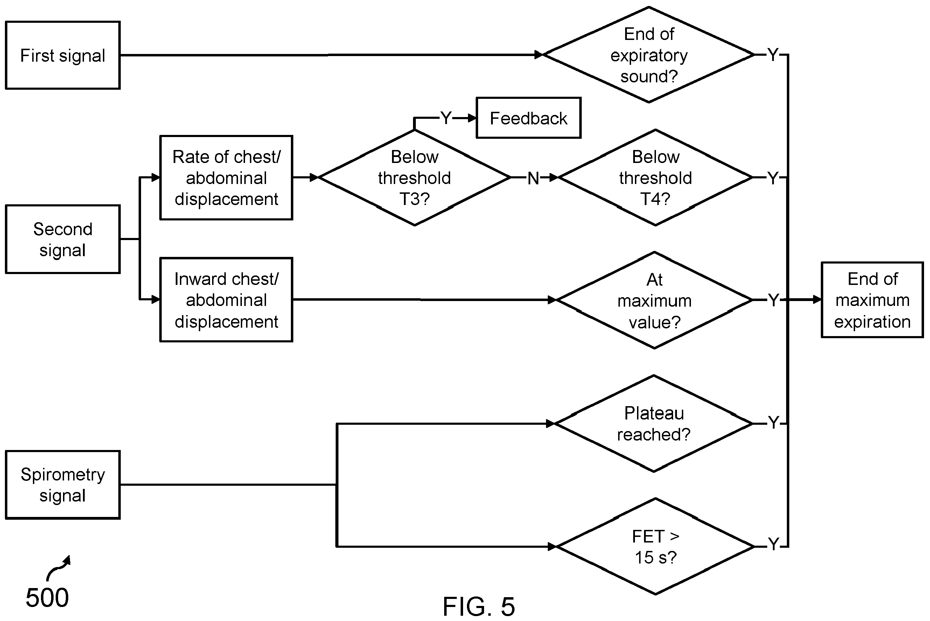

Figure 5 illustrates an example decision tree 500 that may be used to provide guidance during expiration, according to an embodiment of the invention.

A slow start to expiration is detected in response to a determination that a rate of chest and/or abdominal wall displacement is below a third threshold, T3, and feedback is provided to the subject and/or operator, e.g. to prompt the subject to stop the maneuver. Maximum expiration is detected in response to a determination that expiratory sounds have ceased, a determination that a rate of chest and/or abdominal wall displacement is below a fourth threshold, T4, a determination that an inward chest and/or abdominal wall displacement has reached a maximum value, a determination that a plateau has been reached in the spirometry signal, and/or a determination that a forced expiratory time (FET) exceeds 15 seconds (or some other appropriate threshold).

In some examples, instances of early termination of breath (often caused by a subject thinking they have fully exhaled when they have not) may be further reduced by providing guidance throughout expiration (until maximum expiration is reached) to prompt the subject to continue exhaling.

Returning to Figure 1, the processing system 120 may generate guidance information comprising an indication to guide the subject 110 to continue to exhale in response to determining that the conditions for detecting maximum expiration have not been met.

For example, the processing system 120 may generate an indication to guide the subject 110 to continue to exhale in response to a determination that expiratory sounds have not ceased, a determination that a rate of chest and/or abdominal wall displacement is not below a predetermined threshold, a determination that an inward chest and/or abdominal wall displacement has not reached a maximum value, a determination that a plateau has not been reached in the spirometry signal and/or a determination that a forced expiratory time does not exceed a predetermined threshold (e.g. 15 seconds).

An indication to guide the subject 110 to continue to exhale may be provided to the subject and/or operator continuously or at predetermined intervals (e.g. every second) until maximum expiration is reached.

If a subject does terminate their breath before complete exhalation, the spirometry measurements obtained during the maneuver will be faulty. The system 100 may detect early terminations of breath and provide an indication when an early termination of breath is detected, to allow maneuvers in which breath is terminated early to be disregarded.

The processing system 120 may detect an early termination of breath based on the respiratory sounds from the first signal 135, the rate of chest and/or abdominal wall displacement, the inward chest and/or abdominal wall displacement and/or data from the spirometry signal 175.

For example, the processing system 120 may detect an early termination of breath in response to detecting an abrupt end to expiratory sounds in the first signal 135 and/or determining that a rate of chest and/or abdominal wall displacement is below a predetermined threshold (e.g. close to zero), while also determining that a plateau has not been reached in the spirometry signal 175, determining that a forced expiratory time does not exceed a predetermined threshold (e.g. 15 seconds), and/or determining that an inward chest and/or abdominal wall displacement has not reached a maximum value. In other words, the processing system 120 may detect an early termination of breath in response to detecting that the subject 110 has stopped exhaling but does not exhibit other characteristics of maximal expiration. A suitable threshold value of a rate of chest and/or abdominal wall displacement for detecting early termination of breath may be determined based on experimental data.

In some examples, the processing system 120 may detect errors (e.g. coughs, jerky movements and/or subject fatigue) in a spirometry maneuver based on a comparison of the first signal 135 and the second signal 145 with respective first and second reference signals and/or previous signals from the subject 110.

As described above, the processing system 120 may detect coughs and other abnormal movements during a spirometry maneuver by monitoring the posture of the subject 110. The processing system 120 may additionally or alternatively detect coughs and other abnormal movements by comparing the first signal 135 and the second signal 145 with reference signals representative of respiratory sounds and movements, orientations and/or positions of a chest and/or abdominal wall during normal and abnormal chest and/or abdominal wall movements. For example, the processing system 120 may use a mathematical model developed on a database of normal and abnormal chest and/or abdominal wall movements to detect abnormal movements.

In response to detecting a cough or other abnormal movement, the processing system 120 may control the vibrator 161 and/or display device 162 to provide an indication of a detection of an abnormal movement. This allows maneuvers in which abnormal maneuvers occur to be abandoned.

In a spirometry test, a subject performs a plurality of maneuvers so that a "best" maneuver may be selected for final analysis. This may cause the subject to become fatigued and use less than maximal effort when performing later maneuvers, leading to errors in spirometry measurements.

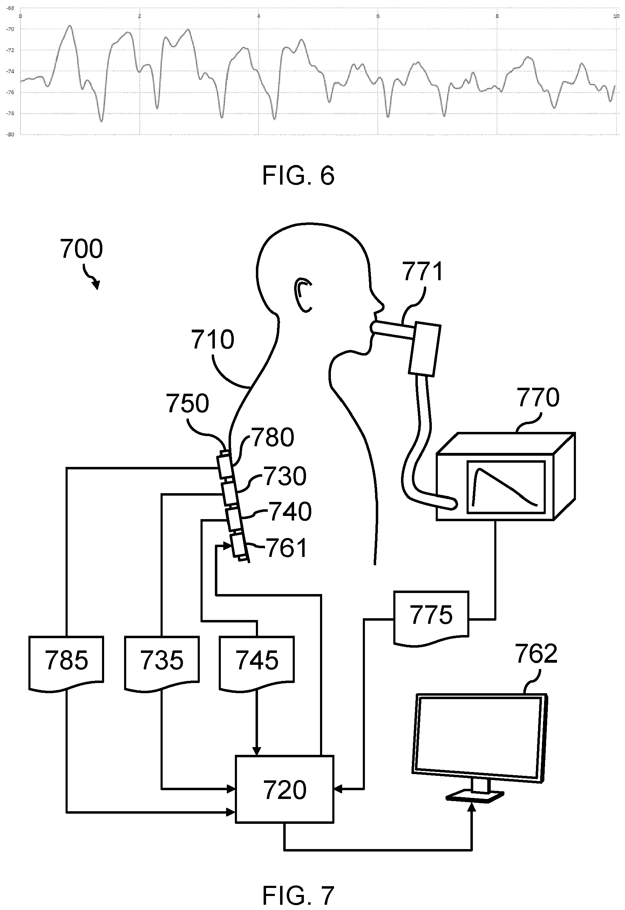

Figure 6 illustrates example IMU (roll in degrees) data from a chest of a subject over multiple respiratory maneuvers. Figure 6 shows how an amplitude of a signal responsive to chest wall movements decreases as the subject becomes fatigued.

Returning to Figure 1, the processing system 120 may detect subject fatigue based on a comparison of one or more characteristics of the first signal 135 and/or second signal 145 during a current maneuver with corresponding characteristics from one or more previous maneuvers. The one or more characteristics may, for example, include amplitude, duration, rate of chest and/or abdominal wall displacement, inward chest and/or abdominal wall displacement, and/or outward chest and/or abdominal wall displacement.

In response to detecting subject fatigue, the processing system 120 may control the vibrator 161 and/or the display device 162 to provide an indication of a detection of subject fatigue.

The "best" maneuver selected for final analysis may be defined based on clinical guidelines, such as those set out in the American Thoracic Society and European Respiratory Society Technical Statement. For example, a "best" maneuver of a plurality of maneuvers may be defined as a maneuver that contains no errors and has a highest value of one or more spirometry parameters (e.g. forced expiratory volume in the first second, FEV1, forced vital capacity, FVC, etc.). A "best" maneuver is conventionally selected based only on spirometry data. Since errors in spirometry recordings are not always evident from the spirometry data, this can lead to maneuvers containing errors being used for final analysis.

The system 100 may be used to improve selection of a "best" maneuver from a spirometry test, by using the first signal 135 and second signal 145 in addition to the spirometry signal 175.

For example, the processing system 120 may process the first signal 135, the second signal 145 and the spirometry signal 175 to select a maneuver of a plurality of maneuvers in the spirometry signal that most closely matches a predetermined desired maneuver. The predetermined desired maneuver may be a maneuver performed by a subject having a desired posture and during which no errors occurred in the spirometry recording.

The processing system 120 may, for example, select the maneuver by analyzing the first signal and second signal received during each of the plurality of maneuvers to identify any errors (such as abnormal movements, inappropriate posture, early termination, subject fatigue, etc.). Errors may be identified using any of the methods described above, or any other suitable method.

The processing system 120 may then define a subset of maneuvers for which no errors have been identified, and select the maneuver from the subset (e.g. based on one or more parameters of the spirometry signal 175). For example, a maneuver may be selected from the subset based on one or more of: a forced expiratory volume in the first second (FEV1), a forced vital capacity (FVC), a back-extrapolated volume (BEV), a forced inspiratory vital capacity (FIVC), a peak expiratory flow (PEF), a forced expiratory time (FET). For instance, the maneuver selected from the subset may be a maneuver with a largest value of FEV1, FVC and/or a sum of FEV1 and FVC. In some examples, more than one maneuver may be selected (e.g. a maneuver with a largest value of FEV1 and a maneuver with a largest value of FVC).

Alternatively, the first signal 135, second signal 145 and spirometry signal 175 may be used to define a subset of maneuvers that meet acceptability and/or usability criteria of a predefined clinical guideline (e.g. the criteria set out in the American Thoracic Society and European Respiratory Society Technical Statement), and an average value for one or more spirometry parameters based on the subset may be used for final analysis.

Figure 7 illustrates a system 700 for providing guidance to a subject 710 and/or operator (not shown) during a spirometry test, according to another embodiment of the invention. The system 700 is similar to the system 100 illustrated in Figure 1, but the patch 750 of system 700 includes at least one electromyography (EMG) sensor 780 in addition to at least one microphone 730, at least one sensor 740 configured to obtain a signal responsive to a movement, orientation and/or position of a chest wall and/or abdominal wall of the subject, and vibrator 761. The at least one electromyography sensor 780 is configured to obtain a signal responsive to an electrical activity of a muscle (e.g. a chest or back muscle) of the subject 710.

Processing system 720, which may be the same as processing system 120 in Figure 1, receives a third signal 785 responsive to an electrical activity of a muscle of the subject from the at least one electromyography sensor 780 in addition to first signal 735 responsive to respiratory sounds of the subject and second signal 745 responsive to a movement, orientation and/or position of a chest wall and/or abdominal wall of the subject.

The processing system 720 generates guidance information by processing the first signal 735, the second signal 745 and the third signal 785, and controls the vibrator 761 and display device 762 to provide a user-perceptible output representative of the generated guidance information. The processing system may process the first and second signal (and optionally spirometry signal 775) using any of the methods described above.

In some examples, the processing system 720 may process the third signal 785 to determine a peak force and/or total force generated by the muscle. The electrical activity of a muscle is correlated with force, and EMG measurements during maximal voluntary contraction are commonly used to determine a measure of the peak force and total force generated by a muscle.

The determined peak force and/or total force generated by the muscle may be used to detect muscle fatigue. Muscle fatigue is associated with a decrease in peak force and total force generated. The processing system 720 may, for example, detect muscle fatigue in response to a determination that the peak force and/or total force is below a predetermined threshold, or based on a comparison with a value for peak force and/or total force from one or more previous maneuvers in the spirometry test.

In response to a detection of muscle fatigue, the processing system 720 may control the vibrator 761 and/or display device 762 to provide an indication of a detection of muscle fatigue to the subject and/or operator. This allows maneuvers that cannot provide useful spirometry measurements due to muscle fatigue to be abandoned.

Additionally or alternatively, a detection of muscle fatigue may be used in the selection of a "best" maneuver from a plurality of maneuvers to be used in final analysis. For example, the processing system 720 may define a subset of maneuvers for which no errors have been identified, as described above, where maneuvers in which muscle fatigue has been detected are excluded from the subset. In another example, the processing system may provide a quality grade for each of the plurality of maneuvers based on the determined peak force and/or total force, and the quality grade may be used alongside spirometry measurements to select a "best" maneuver.

In some examples, the processing system 720 may process the third signal 785 to determine a total muscle mass used during a maneuver. Higher muscle mass recruitment indicates a more forceful effort, so the total muscle mass may be used alongside spirometry measurements in selecting a "best" maneuver of a plurality of maneuvers. The total muscle mass used may also be used as a parameter for comparing different spirometry records (e.g. from different spirometry tests of a subject over time, or from different subjects).

Spirometry is used to provide information on the pulmonary function of both lungs together. However, spirometry measurements alone cannot provide information on specific pathologies. On the other hand, auscultation data (e.g. data from the at least one microphone) provides information on local pathologies but not pulmonary function. Data representative of chest and/or abdominal wall movements may also be used to aid diagnosis of respiratory pathologies. As described above, a breathing pattern may be determined from such data. Some respiratory diseases have characteristic breathing patterns (e.g. Cheyne-Stokes respiration, Kussmaul breathing, etc.) that may be used to diagnose the disease.

The processing system 120 or 720 may process the first signal responsive to respiratory sounds of the subject, the second signal responsive to a movement, orientation and/or position of a chest wall and/or abdominal wall of the subject, and the spirometry signal to provide real-time differential diagnosis of respiratory pathologies.

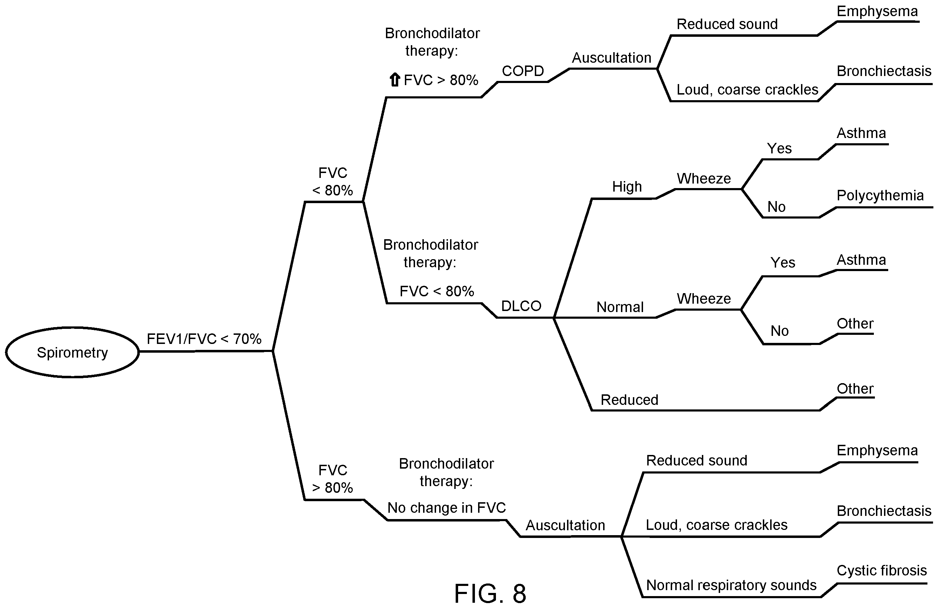

Figure 8 illustrates an example assessment of respiratory pathologies based on spirometry and auscultation data.

In the assessment of Figure 8, an FEV1/FVC ratio (the ratio of the forced expiratory volume in the first second to the forced vital capacity) of less than 70% indicates a respiratory pathology.

Subjects having an FEV1/FVC ratio of less than 70% may be divided into two categories: those with a forced vital capacity (FVC) of less than 80% of a predicted value (e.g. based on the subject's sex, age and ethnicity), and those with an FVC of more than 80% of a predicted value.

For subjects with an FVC of less than 80% of a predicted value, an increase in FVC to above 80% of the predicted value following bronchodilator therapy indicates a chronic obstructive pulmonary disease (COPD). Auscultation data may then be used to determine a particular form of COPD: a reduced sound indicates emphysema, while loud and coarse crackles indicate bronchiectasis.

If a subject's FVC remains below 80% of a predicted value following bronchodilator therapy, this may indicate asthma or another condition. A measurement of the diffusing capacity of the lungs for carbon monoxide (DLCO) and auscultation data may be used to determine which condition. A high or normal DLCO and wheezing in the auscultation data indicates asthma; a high DLCO but no wheezing indicates polycythemia; a normal DLCO but no wheezing indicates another condition (e.g. pulmonary embolism); and a reduced DLCO indicates other conditions (e.g. restrictive lung disease, cardiac insufficiency, etc.).

For subjects with an FVC of more than 80% of a predicted value, no change in FVC following bronchodilator therapy may indicate COPD or cystic fibrosis. Auscultation data may be used to identify subjects having COPD: a reduced sound indicates emphysema, while loud and coarse crackles indicate bronchiectasis. Normal respiratory sounds in the auscultation data indicate cystic fibrosis.

Other appropriate assessments for diagnosing respiratory conditions based on spirometry data and auscultation data will be apparent to the skilled person and may be based on suitable clinical guidelines, such as those published by the UK National Institute for Health and Care Excellence (NICE), the British Thoracic Society (BTS), the European Respiratory Society (ERS), the American College of Physicians (ACP), the American College of Chest Physicians (CHEST), the American Thoracic Society (ATS), and the Global Initiative for Chronic Obstructive Lung Disease (GOLD).

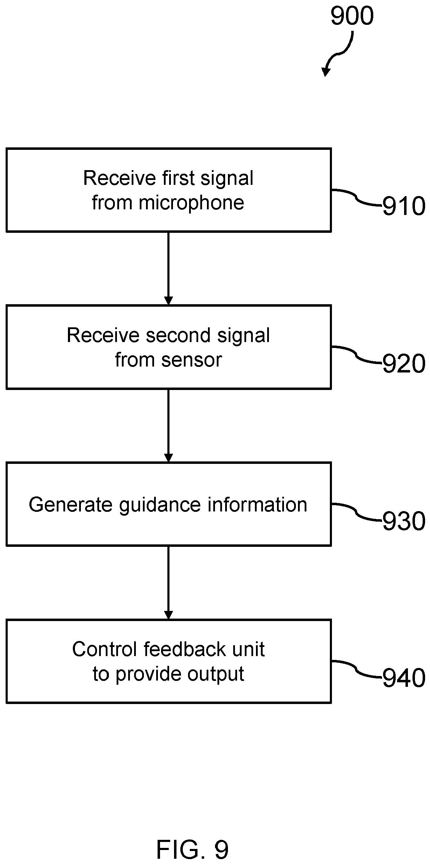

Figure 9 illustrates a computer-implemented method 900 for providing guidance to a subject and/or operator during a spirometry test, according to an embodiment of the invention.

The method begins with step 910, in which a first signal responsive to respiratory sounds of the subject is received from at least one microphone.

At step 920, a second signal responsive to a movement, orientation and/or position of a chest wall and/or abdominal wall of the subject is received from at least one sensor mounted on the subject.

At step 930, guidance information for adjusting one or more characteristics or actions of the subject for the spirometry test is generated by processing the first and second signals.

At step 940, a feedback unit is controlled to provide a user-perceptible output representative of the generated guidance information.

It will be understood that the disclosed methods are computer-implemented methods. As such, there is also proposed a concept of a computer program comprising code means for implementing any described method when said program is run on a processing system.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FGPAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing system (120, 720) for providing guidance to a subject (110, 710) and/or operator during a spirometry test, the processing system being configured to:
receive, from at least one microphone (130, 730), a first signal (135, 735) responsive to respiratory sounds of the subject;
receive, from at least one sensor (140, 740) mounted on the subject, a second signal (145, 745) responsive to a movement, orientation and/or position of a chest wall and/or abdominal wall of the subject;
generate, by processing the first and second signals, guidance information for adjusting one or more characteristics or actions of the subject for the spirometry test; and
control a feedback unit (161, 162, 761, 762) to provide a user-perceptible output representative of the generated guidance information.

2. The processing system (120, 720) of claim 1, wherein the processing system is configured to generate guidance information by performing a process comprising at least one of:
determining a current posture of the subject (110, 710); determining a difference between the current posture of the subject and a desired posture; detecting a start of inspiration; detecting a peak of inspiration; detecting an end of inspiration; determining a rate of chest wall displacement; detecting outward chest displacement; determining a maximum value of outward chest displacement;
determining a rate of abdominal wall displacement; determining an amplitude of abdominal wall displacement; detecting a start of expiration; determining a duration between the peak of inspiration and a start of expiration; determining a maximum inward chest displacement; detecting a peak of expiration; detecting an end of expiration; comparing the received signals (135, 145, 735, 745) with reference signals; comparing the received signals with previous signals from the subject; and/or detecting an early termination of breath.

3. The processing system (120, 720) of claim 1 or 2, wherein the guidance information comprises at least one of: an indication of whether or not the subject (110, 710) has a desired posture; feedback to guide the subject to a desired posture; an indication to prompt the subject to start inspiration; an indication of a detection of a slow start to inspiration; an indication of a detection of a pause at a peak of inspiration; an indication to prompt the subject to start expiration; an indication of a slow start to expiration; an indication to guide the subject to continue to exhale; an indication of a detection of an end of expiration; an indication of a detection of a cough; an indication of a detection of subject fatigue; and/or an indication of a detection of an early termination of breath.

4. The processing system (120, 720) of any of claims 1 to 3, wherein the processing system is further configured to receive a spirometry signal (175, 775) of the subject (110, 710) from a spirometer (170, 770).

5. The processing system (120, 720) of claim 4, wherein the processing system is further configured to analyze the spirometry signal (175, 775) to detect whether the spirometry signal has reached a plateau and/or to calculate a forced expiratory time.

6. The processing system (120, 720) of claim 4 or 5, wherein:
the spirometry signal (175, 775) is representative of a plurality of maneuvers performed by the subject (110, 710), each maneuver comprising a different inspiration and expiration; and
the processing system is further configured to select a maneuver from the plurality of maneuvers that most closely matches a predetermined desired maneuver by processing the spirometry signal and the received first and second signals (135, 145, 735, 745).

7. The processing system (120, 720) of claim 6, wherein the processing system is configured to select the maneuver by:
analyzing, for each maneuver in the spirometry signal (175, 775), the first and second signals (135, 145, 735, 745) received during the maneuver to identify whether any errors occur in the spirometry test performed during the maneuver;
defining a subset of the plurality of maneuvers, wherein the subset comprises only those maneuvers for which no errors are identified by the analysis; and
selecting the maneuver from the subset based on one or more characteristics of the spirometry signal.

8. The processing system (120, 720) of any of claims 4 to 7, wherein the processing system is further configured to provide a diagnosis of respiratory pathology based on the spirometry signal (175, 775) and the received first and second signals (135, 145, 735, 745).

9. The processing system (120, 720) of any of claims 1 to 8, wherein the processing system is further configured to:
receive a third signal (785) responsive to an electrical activity of a muscle of the subject (110, 710) from at least one electromyography sensor (780); and
generate the guidance information by processing the first, second and third signals (135, 145, 735, 745, 785).

10. The processing system (120, 720) of claim 9, wherein the processing system is configured to generate the guidance information by performing a process comprising:
determining a peak force and/or total force generated by the muscle based on the third signal (785); and
detecting muscle fatigue based on the determined peak force and/or total force generated by the muscle.

11. The processing system (120, 720) of claim 9 or 10, wherein the processing system is further configured to determine a total muscle mass used during a maneuver based on the third signal (785).

12. A system (100, 700) for providing guidance to a subject (110, 710) and/or operator during a spirometry test, the system comprising:
at least one microphone (130, 730) configured to obtain a signal responsive to respiratory sounds of the subject;
at least one sensor (140, 740) mounted on the subject configured to obtain a signal responsive to a movement, orientation and/or position of a chest wall and/or abdominal wall of the subject;
the processing system (120, 720) of any of claims 1 to 11, configured to receive the first signal (135, 735) from the at least one microphone and the second signal (145, 745) from at least one position sensor; and
a feedback unit (161, 162, 761, 762), configured to provide a user-perceptible output representative of the generated guidance information based on an instruction from the processing system.

13. The system (100, 700) of claim 12, wherein at least one sensor (140) mounted on the subject comprises at least one inertial measurement unit, at least one accelerometer, and/or at least one strain gauge.

14. A computer-implemented method (900) for providing guidance to a subject (110, 710) and/or operator during a spirometry test, the computer-implemented method comprising:
receiving, from at least one microphone (130, 730), a first signal (135, 735) responsive to respiratory sounds of the subject;
receiving, from at least one sensor (140, 740) mounted on the subject, a second signal (145, 745) responsive to a movement, orientation and/or position of a chest wall and/or abdominal wall of the subject;
generating, by processing the first and second signals, guidance information for adjusting one or more characteristics or actions of the subject for the spirometry test; and
controlling a feedback unit (161, 162, 761, 762) to provide a user-perceptible output representative of the generated guidance information.

15. A computer program product comprising computer program code means which, when executed on a computer device having a processing system, cause the processing system to perform all of the steps of the method (900) according to claim 14.
